# EUROPEAN PATENT APPLICATION

(11) **EP 4 778 782 A1**
(43) Date of publication of application: **22.07.2026**
(21) Application number: 26151805.4
(22) Date of filing: 14.01.2026
(51) Int. Cl.: B60N 2/56, B60R 7/10

(54) **CLOTHING CARE SYSTEM FOR VEHICLE**

(30) Priority: 20.01.2025 KR 20250008232
(71) Applicant: Daechang Seat Co., Ltd.-Dongtan, Hwaseong-si, Gyeonggi-do 18487 (KR)
(72) Inventor: CHO, Chan Ki, 25305 Pyeongchang-gun, Gangwon-do (KR); PARK, Gun Young, 17041 Yongin-si, Gyeonggi-do (KR); HWANG, Bum Jin, 07584 Seoul (KR); PARK, Mun Su, 18119 Osan-si, Gyeonggi-do (KR); HA, Hong Jun, 18127 Osan-si, Gyeonggi-do (KR); JO, Tae Sang, 31158 Cheonan-si, Chungcheongnam-do (KR)
(74) Representative: Stöckeler, Ferdinand

(57) **Abstract**

Proposed is a clothing care system for a vehicle that can not only store clothing easily inside a vehicle but also care for the clothing efficiently. A clothing care system for a vehicle according to an embodiment includes a seat including a backrest having a blower therein and having a vent formed on a rear surface and connected to the blower, and a magnetic coupling portion provided around the vent, and a hanger including a hanger part having an inlet formed in a body and an outlet formed in legs, and a coupling portion provided around the inlet and coupled to the magnetic coupling portion.

## Description

### BACKGROUND

### Technical Field

The present disclosure relates to a clothing care system for a vehicle. More specifically, the present disclosure relates to a clothing care system for a vehicle that can care for clothing hung on a hanger that is hung on the rear surface of a seat of a vehicle.

### Description of the Related Art

In general, driving while wearing a suit jacket or a coat causes considerable inconvenience to a driver, so the driver often removes and stores clothing on the front passenger seat or the rear seat. Meanwhile, when clothing is carelessly stored on the front passenger seat or the rear seat, the clothing may be wrinkled or damaged, so the clothing is hung on a hanger and then hung on an assist handle of the vehicle for storage. However, in this case, there is a drawback that clothing hung on the assist handle obstructs the driver's view and another drawback that the hanger falls off the assist handle due to driving.

Korean Patent Application Publication No. 10-2019-0111449 discloses a clothing deodorizing device for a vehicle.

The clothing deodorizing device for a vehicle disclosed in the publication includes: an air-conditioning unit that forcibly supplies air toward a local area in the vehicle interior; a flow-path opening/closing adjustment unit that adjusts opening and closing of a flow path of the air-conditioning unit; a purification unit that removes odors from an article located in the local area; a control unit that controls operations of the air-conditioning unit, the flow-path opening/closing adjustment unit, and the purification unit; and a hanger part that is installed in the local area and on which clothing is hung.

The clothing deodorizing device for a vehicle has the problem that it lacks a sufficient structure for securing the hanger part to the local area, resulting in weak securing force, and also suffers from low airflow pressure because the airflow path leading to the hanger part is long.

### [Prior Art Document]

### [Patent Document]

(Patent Document 1) KR 10-2019-0111449 B1 (published on October 2, 2019)

### SUMMARY

Proposed is a clothing care system for a vehicle that can not only store clothing easily inside a vehicle but also care for the clothing efficiently.

A clothing care system for a vehicle according to an embodiment includes: a seat including a backrest having a blower therein and having a vent formed on a rear surface and connected to the blower, and a magnetic coupling portion provided around the vent; and a hanger including a hanger part having an inlet formed in a body and an outlet formed in legs, and a coupling portion provided around the inlet and coupled to the magnetic coupling portion.

Further, the vent of the clothing care system for a vehicle according to an embodiment may be opened and closed by a plurality of blades installed in parallel in an up-down direction.

Further, the seat of the clothing care system for a vehicle according to an embodiment may further include a controller provided on a rear surface of the backrest and configured to control the plurality of blades.

Further, the hanger of the clothing care system for a vehicle according to an embodiment may further include: a sterilizing lamp provided on the leg; a heating wire provided inside the hanger part; and a power terminal provided on the body for supplying power and transmitting and receiving signals.

Further, a clothing care system for a vehicle according to another embodiment includes: a seat including a backrest having a vent formed on a rear surface and a magnetic coupling portion provided around the vent; and a hanger including a hanger part having an inlet formed in a body and an outlet formed in legs, and a coupling portion provided around the inlet and coupled to the magnetic coupling portion, wherein the coupling portion of the hanger is formed to protrude, wherein the seat further includes: a first window having a plurality of slats spaced apart in parallel in an up-down direction and installed in contact with the vent; a second window having a plurality of slats spaced apart in parallel in the up-down direction, installed to be slidable in the up-down direction in surface contact with the first window, and having an inclined surface formed at an upper end to incline toward the first window; and an elastic member provided at a lower end of the second window, and wherein the vent has: an open mode in which, when the hanger is coupled to the seat, the second window is slid downward by the coupling portion and the vent is opened; and a closed mode in which, when the hanger is separated from the seat, the second window is slid upward by the elastic member and the vent is closed.

In the clothing care system for a vehicle according to an embodiment, the hanger is magnetically coupled to the seat. As a result, the hanger is firmly coupled and is easily attached and detached.

Further, the clothing care system for a vehicle according to an embodiment has an effect of conveniently and cleanly caring for clothing using air that is blown from the hanger, the heating wire provided inside the hanger, and the sterilizing lamp provided on the leg of the hanger.

Further, in the clothing care system for a vehicle according to an embodiment, the blower provided inside the backrest can supply air toward the front side and the rear side of the backrest. As a result, the clothing care system for a vehicle has a rear-seat blowing effect and a ventilated-seat effect.

Further, since the clothing care system for a vehicle according to an embodiment has a fragrance in the backrest, it is possible to remove odors of clothing hung on the hanger.

Further, the clothing care system for a vehicle according to an embodiment can prevent foreign substances from entering by closing the vents when it is not used.

Further, the clothing care system for a vehicle according to an embodiment can adjust the air volume of the blower using the controller.

Further, in the clothing care system for a vehicle according to an embodiment, the legs of the hanger are hinge-coupled to the body. As a result, the hanger separated from the seat can be straightened and conveniently stowed.

### BRIEF DESCRIPTION OF THE DRAWINGS

FIG. 1 is a perspective view of a clothing care system for a vehicle according to an embodiment;
FIG. 2 is an exploded view of the clothing care system for a vehicle according to an embodiment;
FIG. 3 is a cross-sectional view of a closed mode of the clothing care system for a vehicle according to an embodiment;
FIG. 4 is a cross-sectional view of an open mode of the clothing care system for a vehicle according to an embodiment;
FIG. 5 is a perspective view of a clothing care system for a vehicle according to another embodiment;
FIG. 6 is an exploded view of the clothing care system for a vehicle according to another embodiment;
FIG. 7 is a cross-sectional view of a closed mode of the clothing care system for a vehicle according to another embodiment; and
FIG. 8 is a cross-sectional view of an open mode of the clothing care system for a vehicle according to another embodiment.

### DETAILED DESCRIPTION

The advantages and features of the present disclosure, and methods of achieving them will be clear by referring to the exemplary embodiments that will be described hereafter in detail with reference to the accompanying drawings. However, the present disclosure is not limited to the exemplary embodiments described hereafter and may be implemented in various ways, and the exemplary embodiments are provided to complete the description of the present disclosure and let those skilled in the art completely know the scope of the present disclosure, and the present disclosure is defined by the claims. Like reference numerals indicate the same components throughout the specification.

### Clothing Care System (10) For Vehicle According To An Embodiment

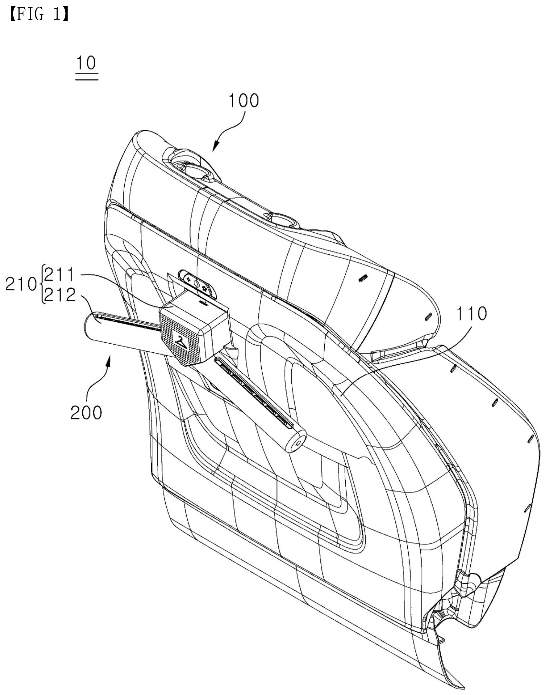

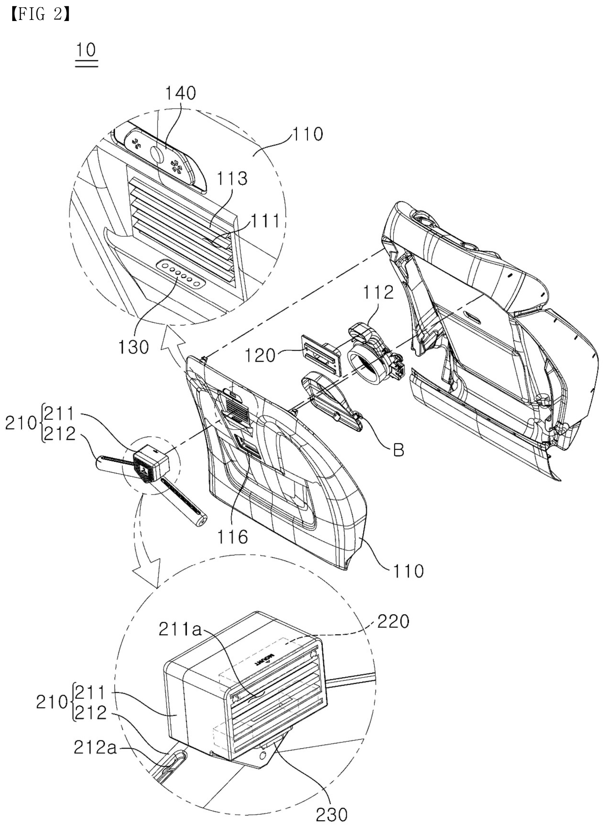

Referring to FIG. 1 that is a perspective view of a clothing care system 10 for a vehicle according to an embodiment (hereafter, referred to as "clothing care system for a vehicle") and FIG. 2 that is an exploded view of the clothing care system 10 for a vehicle, the clothing care system 10 for a vehicle includes a seat 100 and a hanger 200.

The seat 100 includes a backrest 110 and a magnetic coupling portion 120.

The backrest 110 is a part that supports the back of a person seated in the seat 100, and a vent 111 is formed on its rear surface. The vent 111 functions as a passage through which air generated by a blower 112 provided inside the backrest 110 is discharged. Further, an intake hole for supplying air to the blower 112 may be formed at the lower portion of the rear surface of the backrest 110.

The vent 111 can be opened and closed by a plurality of blades 113 installed in parallel in the up-down direction. As an example, the plurality of blades 113 can be configured to rotate in the up-down direction in place and can be rotated manually. In this configuration, when the blades 113 are positioned perpendicular to the ground, the vent 111 is closed, and when the blades 113 are positioned parallel to the ground, the vent 111 is opened.

Further, the blower 112 provided inside the backrest 110 may be configured such that air is also discharged toward the front surface of the backrest 110 by additionally extending a duct for discharging air toward the front of the backrest 110. In this case, since it is possible to discharge air in two directions using one blower 112, both a rear-seat blowing effect and a ventilated-seat effect can be obtained at the same time.

Further, the blower 112 can be firmly fixed inside the backrest 110 using a bracket B.

The magnetic coupling portion 120 is provided around the vent 111. As an example, the magnetic coupling portion 120 may be provided above and below the vent 111.

The hanger 100 is coupled to the rear surface of the seat 100 by the magnetic coupling portion 120, and includes a hanger part 210 and a coupling portion 220.

The hanger part 210 has an inverted-V shape and is composed of a body 211 that corresponds to a central portion and legs 212 that correspond to a leg portion. Further, the legs 212 are hinge-coupled to be rotatable with respect to the body 211, so the hanger 200 can be stowed with minimized volume when it is not used.

An inlet 211a is formed on one side of the body 211 that is coupled to the rear surface of the backrest 110, and an outlet 212a is formed at the lower portion or at the upper and lower portions of the leg 212. That is, when the hanger part 110 is coupled to the backrest 210, air discharged from the vent 111 of the backrest 110 flows into the inlet 211a of the body 111 and is discharged through the outlet 212a of the leg 212.

The coupling portion 220 is provided around the inlet 211a of the body 211. As an example, the coupling portion 220 is provided around the inlet 211a of the body 211 and is configured such that, when it is coupled to a magnetic coupling portion 120, the vent 111 of the backrest 110 and the inlet 211a of the body 211 can communicate with each other.

Further, the seat 100 may further include a controller 140.

The controller 140 is provided on the rear surface of the backrest 110 and can control the plurality of blades 113. As an example, the controller 140 is located around the vent 111, and can adjust opening and closing of the vent 111 by controlling the plurality of blades 113 using an actuator (not shown). When the hanger 200 is coupled to the seat 100, the controller 140 can open the vent 111 by rotating the plurality of blades 113 to be parallel to the ground. On the contrary, when the hanger 200 is separated from the seat 100, the controller 140 can close the vent 111 by rotating the plurality of blades 113 to be perpendicular to the ground.

Further, the controller 140 enables a person seated in the rear seat to use a blowing function by opening the vent 111 by rotating the plurality of blades 113 to be parallel to the ground with the hanger 200 separated from the seat 100.

Further, the controller 140 can adjust the intensity of the airflow that is supplied from the blower 112.

Further, the hanger 200 may further include a sterilizing lamp (not shown), a heating wire (not shown), and a power terminal 230.

The sterilizing lamp is provided on the leg 212 and serves to sterilize the clothing hung on the hanger 200.

The heating wire is provided inside the hanger part 210 and serves to heat the air that is supplied from the vent 111 of the backrest 110.

The power terminal 230 is provided on the body 211 and can be supplied with power for operating the hanger 200 with the hanger 200 coupled to the seat 100. Further, signals for operation control can be transmitted to and received from the seat 200 through the power terminal 230.

Further, the seat 200 has a power terminal 130 that is brought into contact with the power terminal 230 of the hanger 200 to supply power and transmit/receive signals.

Further, the backrest 110 may further include a storage recess 114.

The storage recess 114 is provided on the rear surface of the backrest 110 and is located below the vent 111, and is formed to store a fragrance.

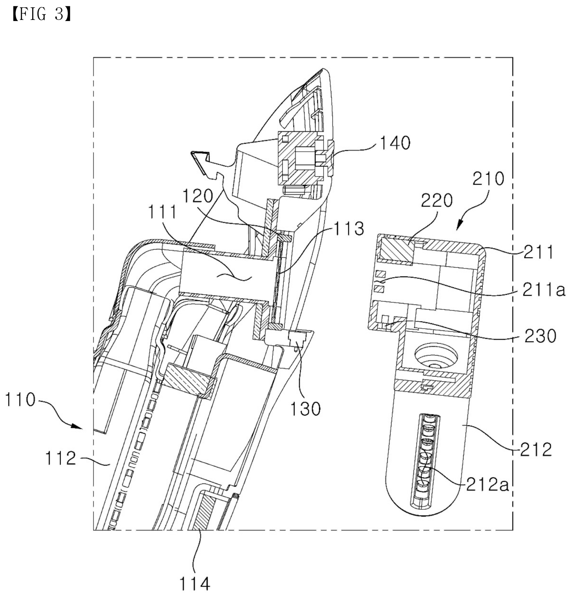

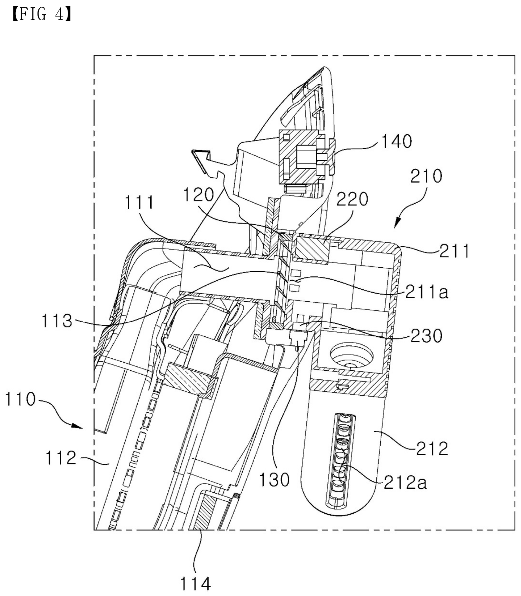

The operation of the clothing care system 10 for a vehicle is described with reference to FIGS. 3 and 4 that illustrate cross-sectional views of the clothing care system 10 for a vehicle.

FIG. 3 illustrates a closed mode of the clothing care system 10 for a vehicle.

The closed mode indicates the state in which the vent 111 is closed and air is not discharged toward the rear surface of the backrest 110.

Specifically, when the coupling portion 220 of the hanger 200 is separated from the magnetic coupling portion 120 of the seat 100, the controller 140 can close the vent 111 by rotating the plurality of blades 113 to be perpendicular to the ground. As a result, when the hanger 200 is not coupled to the seat 100, air can be prevented from being discharged from the vent 111 of the backrest 210.

FIG. 4 illustrates an open mode of the clothing care system 10 for a vehicle.

The open mode indicates the state in which the vent 111 is opened and air supplied from the blower 112 can be discharged to the outlet 212a of the hanger part 210.

Specifically, when the coupling portion 220 of the hanger 200 is coupled to the magnetic coupling portion 120 of the seat 100, the controller 140 enables the vent 111 of the backrest 110 and the inlet 211a of the body 211 to communicate with each other by rotating the plurality of blades 113 to be parallel to the ground. As a result, the air supplied from the blower 112 is discharged to the vent 111 of the backrest 110 and then supplied to the inlet 211a of the body 211, and is discharged to the outlet 212a of the leg 212.

Further, it is also possible to use the system such that air is supplied to a person seated in the rear seat by separating the hanger 100 from the seat 200, manually rotating the plurality of blades 113 to be parallel to the ground, and then making air be discharged from the vent 111 of the backrest 110.

### Clothing Care System (20) For Vehicle According To Another Embodiment

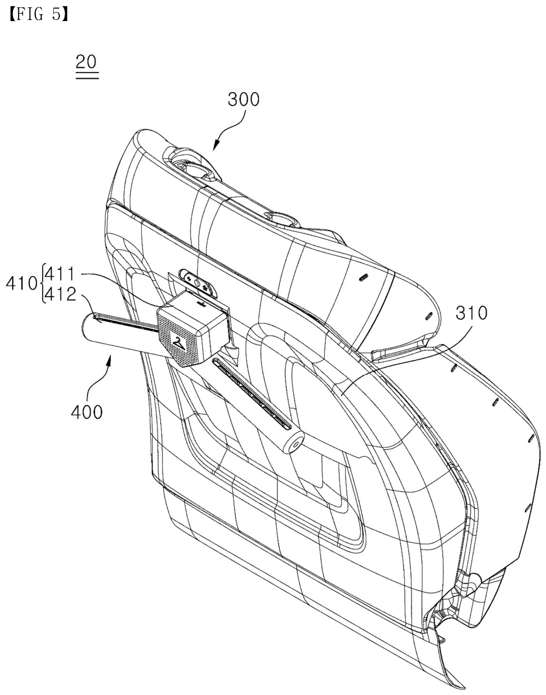

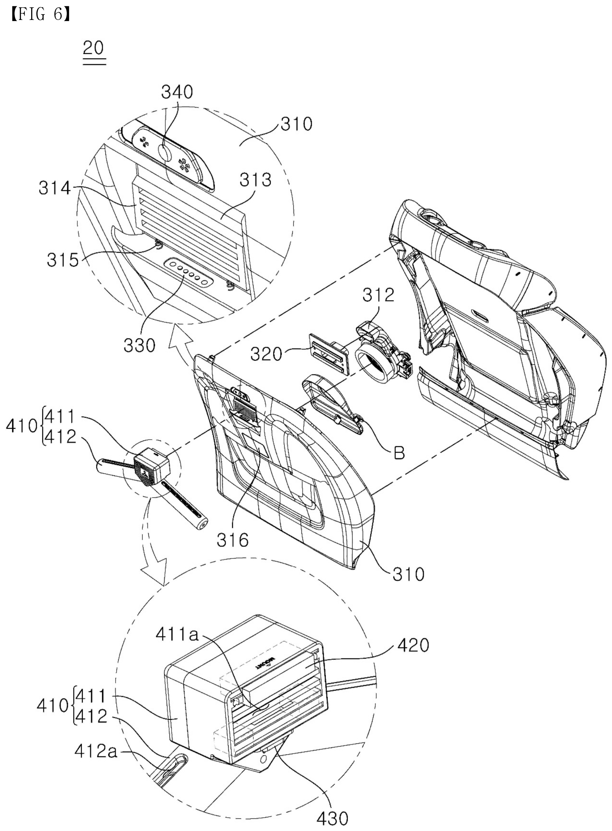

Referring to FIG. 5 that is a perspective view of a clothing care system 20 for a vehicle according to another embodiment (hereafter, referred to as "clothing care system 20 for a vehicle") and FIG. 6 that is an exploded view of the clothing care system 20 for a vehicle, the clothing care system 20 for a vehicle includes a seat 300 and a hanger 400.

The seat 300 includes a backrest 310 and a magnetic coupling portion 320.

The backrest 310 is a part that supports the back of a person seated in the seat 300, and a vent 311 is formed on its rear surface. The vent 311 functions as a passage through which air generated by a blower 312 provided inside the backrest 310 is discharged. Further, an intake hole for supplying air to the blower 312 may be formed at the lower portion of the rear surface of the backrest 310.

The vent 311 can be opened and closed by a first window 313 and a second window 314. As an example, the backrest 310 may include a first window 313, a second window 314, and an elastic member 315.

The first window 313 has a plurality of slats 313a spaced apart in parallel in the up-down direction, and is installed to be in contact with the vent 311. A plurality of discharge holes 313b is formed in the first window 313 by the plurality of slats 313a.

The second window 314 has a plurality of slats 314a spaced apart in parallel in the up-down direction, and is installed to be slidable in the up-down direction in surface contact with the first window 313. A plurality of discharge holes 314b is formed in the second window 314 by the plurality of slats 314a. In this configuration, the plurality of slats 314a of the second window 314 has an area corresponding to the plurality of discharge holes 313b of the first window 313. Further, the second window 314 has, at its upper end, an inclined surface 314c that is inclined toward the first window 313.

When the second window 314 is slid downward such that the plurality of blades 314a of the second window 314 is positioned parallel with the plurality of blades 313a of the first window 313, the vent 311 can be opened.

Further, the elastic member 315 is provided at the lower end of the second window 314 and elastically supports the second window 314. When the second window 314 is slid downward, it can be slid upward again by the elastic member 315.

Further, the blower 312 provided inside the backrest 310 may be configured such that air is also discharged toward the front surface of the backrest 310 by additionally extending a duct for discharging air toward the front of the backrest 310. In this case, since it is possible to discharge air in two directions using one blower 312, both a rear-seat blowing effect and a ventilated-seat effect can be obtained at the same time.

Further, the blower 312 can be firmly fixed inside the backrest 310 using a bracket B.

The magnetic coupling portion 320 is provided around the vent 311. As an example, the magnetic coupling portion 320 may be provided above and below the vent 311.

The hanger 400 is coupled to the rear surface of the seat 300 by the magnetic coupling portion 320, and includes a hanger part 410 and a coupling portion 420.

The hanger part 410 has an inverted-V shape and is composed of a body 411 that corresponds to a central portion and legs 412 that correspond to a leg portion. Further, the legs 412 are hinge-coupled to be rotatable with respect to the body 411, so the hanger 400 can be stowed with minimized volume when it is not used.

An inlet 411a is formed on one side of the body 411 that is coupled to the rear surface of the backrest 310, and an outlet 412a is formed at the lower portion or at the upper and lower portions of the leg 412. That is, when the hanger part 410 is coupled to the backrest 310 and the vent 311 of the backrest 310 and the inlet 411a of the body 411 communicate with each other, air supplied from the blower 312 can be discharged to the outlet 412a of the leg 412.

The coupling portion 420 is formed to protrude around the inlet 411a of the body 411. As an example, the coupling portion 420 is formed to protrude around the inlet 411a of the body 411 and is configured such that, when it is coupled to a magnetic coupling portion 320, the vent 311 of the backrest 310 and the inlet 411a of the body 411 can communicate with each other.

Further, the seat 300 may further include a controller 340.

The controller 340 is provided on the rear surface of the backrest 310 and can slide the second window 314 in the up-down direction. As an example, the controller 340 is located around the vent 311 and can adjust opening and closing of the vent 311 by controlling the position of the second window 314. The controller 340 slides the second window 314 downward such that the discharge holes 313b of the first window 313 and the discharge holes 314b of the second window 314 communicate with each other, whereby the vent 311 can be opened. In this case, a person seated in the rear seat can use the blowing function.

Further, the controller 340 can adjust the intensity of the airflow that is supplied from the blower 312.

Further, the hanger 400 may further include a sterilizing lamp (not shown), a heating wire (not shown), and a power terminal 430.

The sterilizing lamp is provided on the leg 412 and serves to sterilize the clothing hung on the hanger 400.

The heating wire is provided inside the hanger part 410 and serves to heat the air that is supplied from the vent 311 of the backrest 310.

The power terminal 430 is provided on the body 411 and can be supplied with power for operating the hanger 400 with the hanger 400 coupled to the seat 300. Further, signals for operation control can be transmitted to and received from the seat 300 through the power terminal 430.

Further, the seat 300 has a power terminal 330 that is brought into contact with the power terminal 430 of the hanger 400 to supply power and transmit/receive signals.

Further, the backrest 310 may further include a storage recess 316.

The storage recess 316 is provided on the rear surface of the backrest 310 and is located below the vent 311, and is formed to be able to store a fragrance.

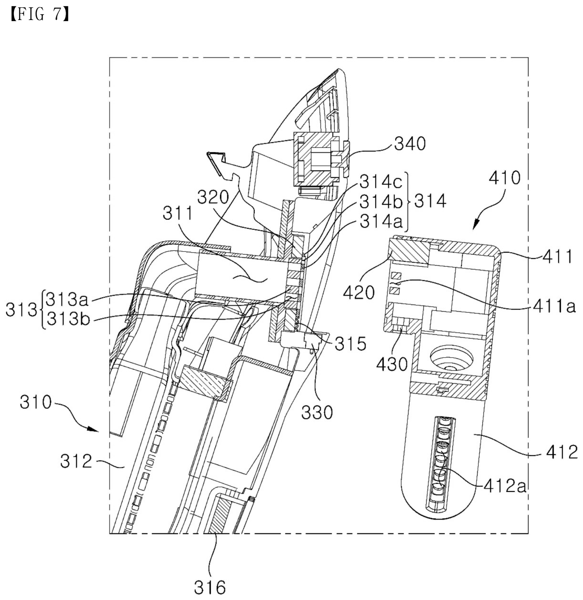

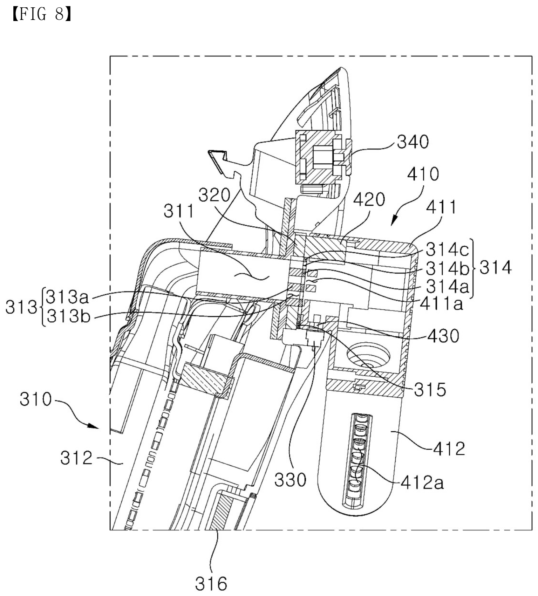

The operation of the clothing care system 20 for a vehicle is described with reference to FIGS. 7 and 8 that illustrate cross-sectional views of the clothing care system 20 for a vehicle.

FIG. 7 illustrates a closed mode of the clothing care system 20 for a vehicle.

The closed mode indicates the state in which the vent 311 is closed and air is not discharged toward the rear surface of the backrest 310.

Specifically, when the coupling portion 420 of the hanger 400 is separated from the magnetic coupling portion 320 of the seat 300, the second window 314 is moved upward by the elastic member 315, whereby the blades 313a of the first window 313 and the blades 314a of the second window 314 are positioned out of alignment. As a result, air can be prevented from being discharged from the vent 311 of the backrest 310.

FIG. 8 illustrates an open mode of the clothing care system 20 for a vehicle.

The open mode indicates the state in which the vent 311 is opened and air supplied from the blower 312 can be discharged to the outlet 412a of the hanger part 410.

Specifically, when the coupling portion 420 of the hanger 400 is coupled to the magnetic coupling portion 320 of the seat 300, the protruding coupling portion 420 pushes the second window 314 downward along the inclined surface 314c of the second window 314. As a result, the second window 314 is moved downward, and the blades 313a of the first window 313 and the blades 314a of the second window 314 are positioned in alignment. That is, the discharge holes 313b of the first window 313 and the discharge holes 314b of the second window 314 communicate with each other, so the air supplied from the blower 312 is discharged to the vent 311 of the backrest 310, supplied to the inlet 411a of the body 411, and then discharged to the outlet 412a of the leg 412.

The expressions used to describe the embodiments of the present disclosure (such as terms and visualized images) are merely selected for the purpose of aiding understanding of the technology.

In addition, for convenience of explanation, the present disclosure has been described with a limited number of embodiments, and those skilled in the art will be able to devise new embodiments on the basis of the described embodiments without departing from a scope of the present disclosure.

Therefore, the claims of the present disclosure should not be limited by certain expressions shown in the "Description of the Invention" and the drawings, and should be interpreted broadly on the basis of the inherent spirit embodied throughout the specification.

## Claims

1. A clothing care system for a vehicle, the system comprising:
a seat including a backrest having a blower therein and having a vent formed on a rear surface and connected to the blower, and a magnetic coupling portion provided around the vent; and
a hanger including a hanger part having an inlet formed in a body and an outlet formed in legs, and a coupling portion provided around the inlet and coupled to the magnetic coupling portion.

2. The clothing care system for a vehicle of claim 1, wherein the vent is opened and closed by a plurality of blades installed in parallel in an up-down direction.

3. The clothing care system for a vehicle of claim 2, wherein the seat further includes a controller provided on a rear surface of the backrest and configured to control the plurality of blades.

4. The clothing care system for a vehicle of claim 1, wherein the hanger further includes:
a sterilizing lamp provided on the leg;
a heating wire provided inside the hanger part; and
a power terminal provided on the body for supplying power and transmitting and receiving signals.

5. The clothing care system for a vehicle of claim 1, wherein the coupling portion of the hanger is formed to protrude,
the seat further includes:
a first window having a plurality of slats spaced apart in parallel in an up-down direction and installed in contact with the vent;
a second window having a plurality of slats spaced apart in parallel in the up-down direction, installed to be slidable in the up-down direction in surface contact with the first window, and having an inclined surface formed at an upper end to incline toward the first window; and
an elastic member provided at a lower end of the second window, and
the vent has:
an open mode in which, when the hanger is coupled to the seat, the second window is slid downward by the coupling portion and the vent is opened; and
a closed mode in which, when the hanger is separated from the seat, the second window is slid upward by the elastic member and the vent is closed.
